# EUROPEAN PATENT APPLICATION

(11) **EP 3 544 020 A1**
(43) Date of publication of application: **25.09.2019**
(21) Application number: 19163699.2
(22) Date of filing: 19.03.2019
(51) Int. Cl.: G16H 50/70, G16H 50/20, G16H 30/40

(54) **AUTOMATIC ANALYSIS OF A LARGE PATIENT POPULATION USING MEDICAL IMAGING DATA**

(30) Priority: 20.03.2018 US 201862645454 P; 20.03.2018 US 201862645450 P
(71) Applicant: Siemens Healthcare GmbH, 91052 Erlangen (DE)
(72) Inventor: Grbic, Sasa, Plainsboro, NJ New Jersey 08536 (US); Georgescu, Bogdan, Plainsboro, NJ New Jersey 08536 (US); Comaniciu, Dorin, Princeton Junction, NJ New Jersey 08550 (US); Yu, Daphne, Yardley, PA 19067 (US); Chabin, Guillaume, Princeton, NJ 08540 (US); Re, Thomas, Monroe, NJ 08831 (US); Ezzi, Afshin, Princeton, NJ 08540 (US)
(74) Representative: Patentanwälte Bals & Vogel

(57) **Abstract**

Systems and methods are provided for determining an analytic measure of a patient population. A knowledge database comprising structured patient data for a patient population is maintained. The structured patient data is generated by processing unstructured medical imaging data for the patient population using one or more machine learning algorithms. An analytic measure of the patient population is determined based on the structured patient data of the knowledge database. The analytic measure of the patient population is output.

## Description

### TECHNICAL FIELD

The present invention relates generally to automatic analysis of a large patient population using medical imaging data, and more particularly to automatically processing medical imaging data using one or more machine learning algorithms to generate quantitative patient data for analyzing a large patient population.

### BACKGROUND

Analyzing large patient populations may reveal medical trends or relationships that can be used for planning, diagnosis, decision support, and intervention. Such patient populations are typically analyzed based on medical images acquired for those patients. With the continued advancement in medical imaging technology, the amount of medical images that is acquired, and the amount of information encapsulated in those medical images, is continuously increasing. Typically, medical images are analyzed by radiologists who interpret the medical images to generate medical imaging reports (e.g., radiology reports) on the medical images. This is a tedious process due to the large amount of medical images, the large amount of information that the medical images encapsulate, the pressure to interpret the medical images quickly and efficiently, the continuous effort required to maintain reading consistency, the lower reimbursement amounts provided by insurance companies, the quality of the medical images, and the reporting requirements. As a result, radiologists interpreting the medical images typically do not extract as much information as is available in the medical images and as such, a large amount of information encapsulated in medical images is not captured in the medical imaging reports in a quantitative format.

In addition, such medical images and medical imaging reports on the medical images are not quantitatively organized in a structured format in a manner to facilitate analysis of the patient population. Specifically, medical images are typically provided as raw image pixels and medical imaging reports are typically provided as free text. While medical images may be conventionally stored according to the DICOM (digital imaging and communications in medicine) standard, DICOM tags are populated before analysis of the medical images by the radiologist. As such, DICOM tags mostly include information relating to scanning procedures and include very little quantitative information relating to the content of the medical images. Similarly, medical imaging reports may be conventionally organized into fields, however fields relating to image interpretation are typically provided as free text. Information from medical images and medical imaging reports are typically partitioned and therefore it is tedious to cross reference such information. Thus, it is difficult to analyze a large patient population where the medical images and the medical imaging reports of the patient population are not in a quantitative structured format.

### BRIEF SUMMARY OF THE INVENTION

One or more embodiments of the present invention provide for automatic processing of unstructured and non-quantitative medical images and/or medical imaging reports of a large patient population to generate structured patient data using one or more machine learning algorithms. Advantageously, analytic measures for the patient population as a whole may be generated by searching, parsing, or otherwise analyzing the structured patient data. Embodiments of the present invention enable the automatic transformation of raw data into new and actionable information that can be used for planning, diagnosis, decision support, and intervention.

According to a first aspect of the invention a method according claims 1 to 7 is provided, in particular a method for automatic analysis of a patient population is provided. In accordance with one or more embodiments, systems and methods are provided for determining an analytic measure of a patient population. A knowledge database comprising structured patient data for a patient population is maintained. The structured patient data is generated by processing unstructured medical imaging data for the patient population using one or more machine learning algorithms. An analytic measure of the patient population is determined based on the structured patient data of the knowledge database. The analytic measure of the patient population is output.

In accordance with one or more embodiments, the unstructured medical imaging data comprises medical images of the patient population and medical imaging reports of the medical images. The structured patient data comprises anatomical structures, one or more segmented anatomical structures, and quantitative patient data. The quantitative patient data may include a presence, a position, a distance, a diameter, a circumference, a volume, a surface area, and/or a score determined from medical records (e.g., patient related information, reconstructed medical images, detected, annotated, or segmented anatomical structures, other quantitative patient data, etc.) of the patient population. The unstructured medical imaging data for the patient population is processed to generate the structured patient data by detecting the anatomical structures in medical images of the unstructured medical imaging data for the patient population, segmenting the one or more of the anatomical structures from the medical images, and determining the quantitative patient data based on the medical records of the patient population.

In accordance with one or more embodiments, the analytic measure of the patient population is determined by generating a graph or a table based on the structured patient data. The analytic measure of the patient population may be in response to a user request. For example, the request may be a query for filtered structured patient data from the knowledge database. The analytic measure of the patient population may be displayed on a display device, or may be exported to a file. According to a second aspect of the invention an method apparatus is provided, the apparatus comprising: means for maintaining a knowledge database comprising structured patient data for a patient population, the structured patient data generated by processing unstructured medical imaging data for the patient population using one or more machine learning algorithms; means for determining an analytic measure of the patient population based on the structured patient data of the knowledge database; and means for outputting the analytic measure of the patient population. Preferred is an apparatus, wherein the structured patient data comprises anatomical structures, one or more segmented anatomical structures, and quantitative patient data. Further, an apparatus is preferred, wherein processing unstructured medical imaging data for the patient population using one or more machine learning algorithms comprises: means for detecting the anatomical structures in medical images of the unstructured medical imaging data for the patient population; means for segmenting the one or more of the anatomical structures from the medical images; and means for determining the quantitative patient data based on medical records of the patient population. Preferred is further an apparatus, wherein the quantitative patient data comprises at least one of a presence, a position, a distance, a diameter, a circumference, a volume, a surface area, and a score.

According to a third aspect of the invention a non-transitory computer readable medium storing computer program instructions apparatus is provided. The computer program instructions when executed by a processor cause the processor to perform operations comprising: maintaining a knowledge database comprising structured patient data for a patient population, the structured patient data generated by processing unstructured medical imaging data for the patient population using one or more machine learning algorithms; determining an analytic measure of the patient population based on the structured patient data of the knowledge database; and outputting the analytic measure of the patient population.

Preferred is a non-transitory computer readable medium storing computer program instructions apparatus, wherein determining an analytic measure of the patient population based on the structured patient data of the knowledge database comprises: enerating a graph or a table based on the structured patient data.

Preferred is further a non-transitory computer readable medium storing computer program instructions apparatus, wherein the determining the analytic measure of the patient population is in response to a user request.

Preferred is further a non-transitory computer readable medium storing computer program instructions apparatus, wherein the unstructured medical imaging data comprises medical images of the patient population and medical imaging reports of the medical images.

Preferred is a non-transitory computer readable medium storing computer program instructions apparatus, wherein outputting the analytic measure of the patient population comprises displaying the analytic measure of the patient population on a display device. The non-transitory computer readable medium storing computer program instructions apparatus is designed to perform a method according to the first aspect of the invention.

These and other advantages of the invention will be apparent to those of ordinary skill in the art by reference to the following detailed description and the accompanying drawings.

### BRIEF DESCRIPTION OF THE DRAWINGS

Figure 1 shows an high-level diagram of a communications system, in accordance with one or more embodiments;
Figure 2 shows a method for determining an analytic measure of a patient population, in accordance with one or more embodiments;
Figure 3 shows a method for processing medical images for a patient population to generate structured patient data, in accordance with one or more embodiments;
Figure 4 shows exemplary structures that may be detected in medical images, in accordance with one or more embodiments;
Figure 5 shows a workflow for detecting anatomical structures in a medical image, in accordance with one or more embodiments;
Figure 6 shows an adversarial deep image-to-image network for segmenting one or more anatomical structures from medical images, in accordance with one or more embodiments;
Figures 7-10 show various plots representing analytic measures of a patient population, in accordance with one or more embodiments;
Figures 11-14 show various user interfaces for generating analytic measures of a patient population, in accordance with one or more embodiments; and
Figure 15 shows a high-level block diagram of a computer.

### DETAILED DESCRIPTION

The present invention generally relates to systems and methods for the automatic analysis of a large patient population using medical imaging data. Embodiments of the present invention are described herein to give a visual understanding of such systems and methods. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Further, it should be understood that while the embodiments discussed herein may be discussed with respect to automatic analysis of a large patient population using medical imaging data, the present invention is not so limited. Embodiments of the present invention may be applied for the analysis of any type of data using any type of imaging data.

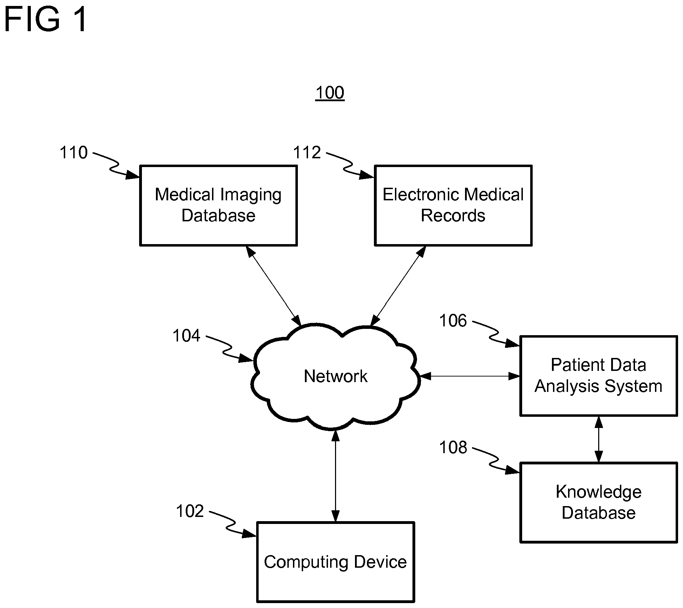

Figure 1 shows a high-level diagram of a communications system 100, in accordance with one or more embodiments. Communications system 100 includes one or more computing devices 102. Computing device 102 may comprise any type of computing device, such as, e.g., a computer, a tablet, or a mobile device. Computing device 102 is operated by end users (e.g., doctors, medical professionals, or any other user) for communicating via network 104. Network 104 may include any type of network or combinations of different types of networks, and may be implemented in a wired and/or a wireless configuration. For example, network 104 may include one or more of the Internet, an intranet, a local area network (LAN), a wide area network (WAN), a cellular communications network, etc.

End users of computing device 102 may communicate via network 104 for interacting with a patient data analysis system 106 and knowledge database 108 to retrieve analytic measures of a large patient population, such as, e.g., a cohort of patients. End users may interact with patient data analysis system 106 and knowledge database 108 via an interface of a web browser executing on computing device 102, an application executing on computing device 102, an app executing on computing device 102, or any other suitable interface for interacting with patient data analysis system 106. In one example, end users of computing devices 102 may interact with a software as a service (SaaS) application hosted by patient data analysis system 106.

Communication system 100 also includes medical imaging database 110 (such as, e.g., a picture archiving and communication system, PACS) for storing medical images of the patient population and electronic medical records 112 for storing other patient related information of the patient population, such as, e.g., medical imaging reports (e.g., radiology reports) of the medical images, as well as demographic information (e.g., age, gender, and weight), medical history, medication and allergies, immunization status, laboratory test results, vital signs, etc.

Conventionally, medical imaging data (e.g., medical images and medical imaging reports of the patient population) is not in a quantitative or structured format. Specifically, medical images are typically provided as raw image pixels and medical imaging reports are typically provided as free text. Medical images encapsulate a large amount of information that is not in a quantitative format. Medical images and medical imaging reports are also typically unstructured in that they are not quantitatively organized in a data structure. It is difficult to search, parse, or otherwise analyze a large patient population to determine analytic measures of the patient population where medical images or medical imaging reports of the patient population are not in a structured or quantitative format.

Patient data management system 106 is configured to automatically process unstructured medical imaging data (e.g., unstructured medical images and/or unstructured medical imaging reports) to extract structured patient data using one or more machine learning algorithms, and store the extracted patient data in knowledge database 108 in a structured format. The one or more machine learning algorithms facilitate the automatic processing of large amounts of patient data for the patient population. Advantageously, analytic measures for the patient population as a whole may be generated by searching, parsing, or otherwise analyzing the structured patient data. Patient data management system 106 enables the automatic transformation of raw data into new and actionable information that can be used for planning, diagnosis, decision support, and intervention.

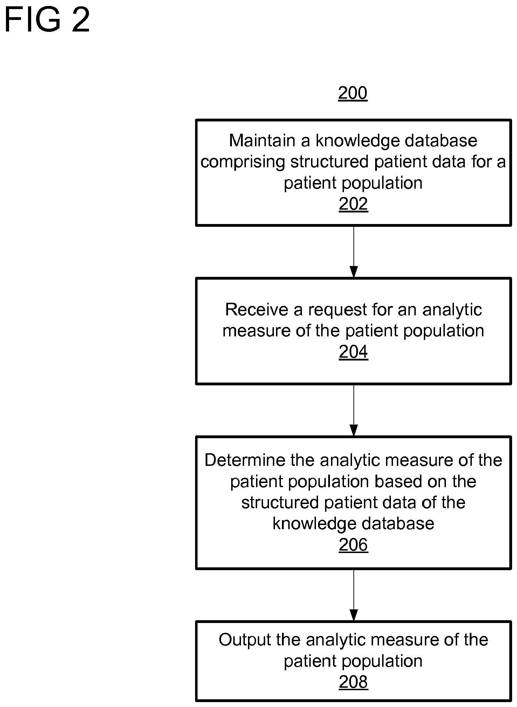

Figure 2 shows a method 200 for determining an analytic measure of a patient population, in accordance with one or more embodiments. Method 200 will be described with reference to communications system 100 of Figure 1. In one embodiment, the steps of method 200 are performed by patient data analysis system 106.

At step 202, a knowledge database (e.g., knowledge database 108) comprising structured patient data for the patient population is maintained. The patient population represents a large group or cohort of patients. The structured patient data is generated by processing unstructured medical imaging data for the patient population using one or more machine learning algorithms. The structured patient data is organized and stored in the knowledge database in one or more data structures. The data structures may be of any suitable format to enable searching, parsing, or otherwise analyzing of the structured patient data. For example, the structured patient data may be organized and stored as one or more arrays, linked lists, tuples, tables, etc.

The structured patient data may comprise any type of data relating to the patient population. For example, the structured patient data may include patient related information (e.g., medical history, demographic information, etc.), medical images reconstructed based on acquisition information (e.g., DICOM information), anatomical structure information (e.g., landmarks, organs, organ sub-structures, systems, etc.) detected in the medical images using a machine learning algorithm, segmented anatomical structures generated using a machine learning algorithm, and/or quantitative patient data. The quantitative patient data is any quantitative measure of interest measured, calculated, or otherwise determined from any medical record. For example, the quantitative patient data may be patient related information, reconstructed medical images (reconstructed based on acquisition information), detected, annotated, or segmented anatomical structures, or other quantitative patient data. Examples of quantitative patient data include a presence or position of an anatomical structure, a distance between two anatomical structures, a volume or surface area of an anatomical structure, a surface of an anatomical structure, a diameter or circumference of an anatomical structure, a score associated with an anatomical structure (e.g., a lesion score), etc.

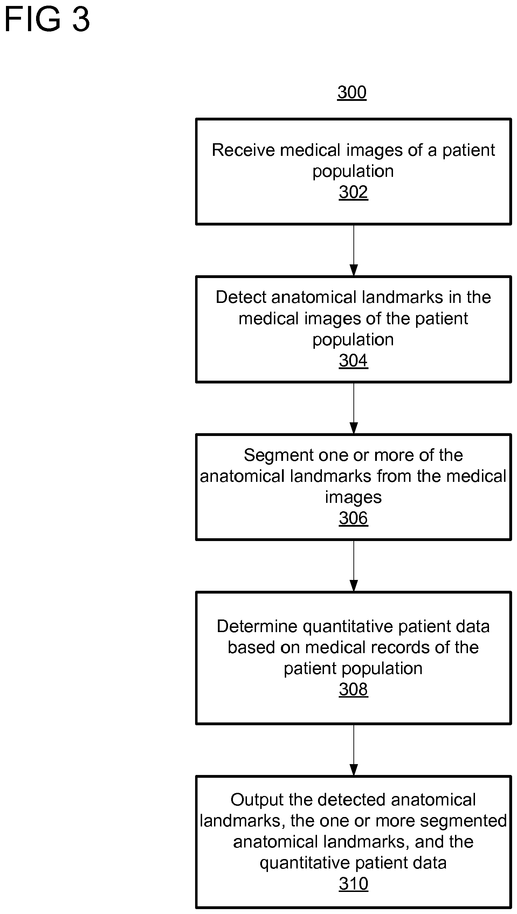

In one embodiment, the unstructured medical imaging data for the patient population is processed to generate the structured patient data by detecting the anatomical structures in medical images of the unstructured medical imaging data for the patient population, segmenting anatomical structures from the medical images, determining the quantitative patient data based segmented anatomical structures, reconstructing medical image acquisition with extraction of scanning metadata present in the medical imaging database 110, extraction of related patient data (e.g., treatment and medical history, diagnosis from reports, etc.) from electronic medical records 112, and non-radiology related data from other hospital information system (HIS) integrating electronic medical records. In one embodiment, the structured patient data is generated by processing medical imaging data for the patient population according to the steps of method 300 of Figure 3, as described in detail below.

At step 204, a request for an analytic measure of the patient population is received. The request may be received from a user, such as, e.g., an end user of computing device 102 of Figure 1. The request may include parameters for determining the analytic measure of the patient population. For example, the parameters may define factors or variables to be analyzed to determine the analytic measure of the patient population. In one example, the request is a query for patient data of the patient population from the knowledge database for filtering. The query may be a custom query for advanced filtering of the patient data.

The analytic measure of the patient population is any measure of interest for analyzing the patient population. In one embodiment, the analytic measure of the patient population is a statistical measure of the patient population. In another embodiment, the analytic measure of the patient population is patient data of the patient population resulting from an analysis (e.g., parsing, filtering, or searching). The analytic measure of the patient population may be of any suitable form. For example, the analytic measure of the patient population may be visually represented, e.g., as a graph, plot, or table, or may be represented as discrete values (e.g., an average, a standard deviation, etc.). Examples of the analytic measure of the patient population may include a table of data parsed or filtered according to the structured patient data and a comparison of two or more factors or variables according to the structured patient data shown in a graph or a plot.

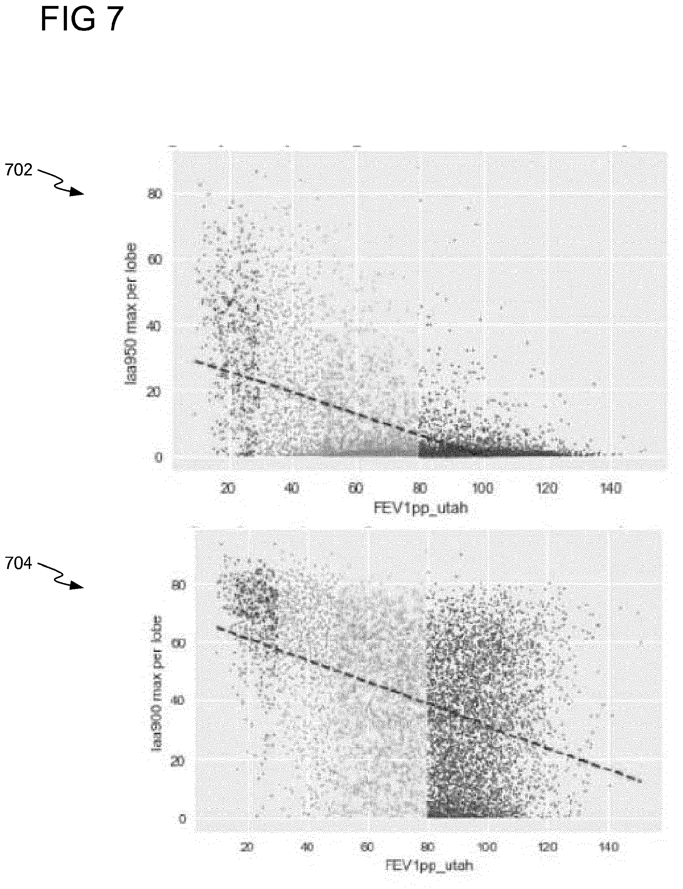

At step 206, in response to receiving the request, the analytic measure of the patient population is determined based on the structured patient data of the knowledge database. In one embodiment, the structured patient data may be analyzed according to the parameters of the request to determine the analytic measure. For example, the structured patient data may be filtered based on an anatomical structure, quantitative patient data, or any other patient data (e.g., age, weight, gender, etc.) as defined by the parameters, and the filtered structured patient data may be represented by generating a table, chart, or any other suitable visual representation as the analytic measure of the patient population. In another example, two or more factors or variables of the structured patient data may be compared with each other as defined by the parameters. The comparison may be represented by generating a table, chart, graph, plot, or any other suitable visual representation as the analytic measure of the patient population. Examples of the analytic measure of the patient population are shown in Figures 7-14, described below.

At step 208, the analytic measure of the patient population is output. In one embodiment, the analytic measure of the patient population may be output by exporting the analytic measure to a file (e.g., a comma-separate values (CSV) file), which may be used as input to other analytic systems or tools for deeper analysis. In other embodiments, the analytic measure of the patient population can be output by displaying the analytic measure of the patient population on a display device of a computer system (e.g., computing system 102), storing the analytic measure of the patient population on a memory or storage of a computer system, or by transmitting the analytic measure of the patient population to a remote computer system.

Figure 3 shows a method 300 for processing medical images for a patient population to generate structured patient data, in accordance with one or more embodiments. The steps of method 300 may be performed to generate the structured patient data maintained in the knowledge database at step 202 of Figure 2.

At step 302, medical images of a patient population is received. The medical images are not in a structured or quantitative format. The medical images may be of any suitable modality, such as, e.g., x-ray, magnetic resonance imaging (MRI), computed tomography (CT), DynaCT (DTC), ultrasound (US), single-photon emission computed tomography (SPECT), positron emission tomography (PET), or any other suitable modality or combination of modalities.

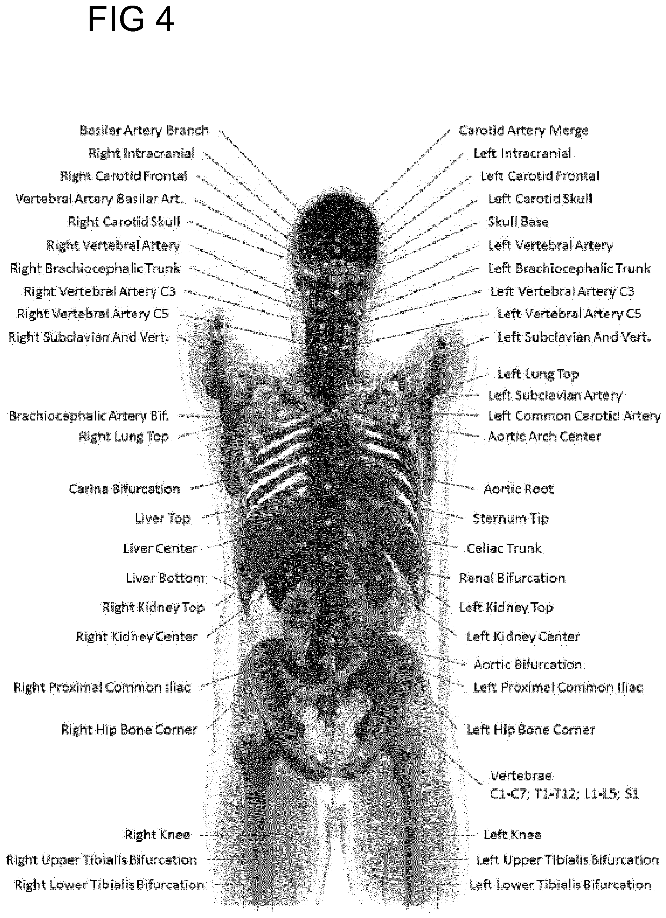

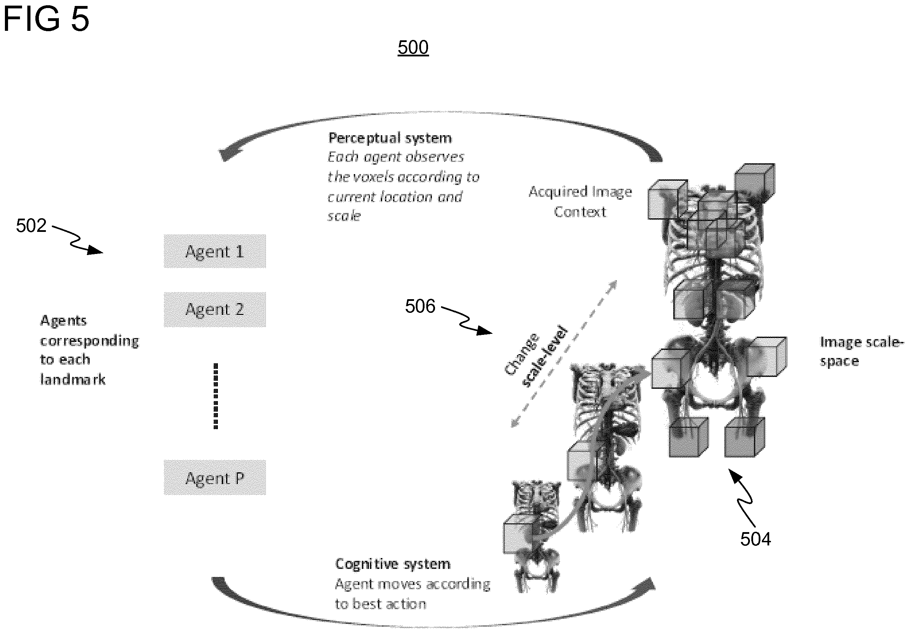

At step 304, anatomical structures are detected in the medical images of the patient population. The anatomical structures may include landmarks, organs, organ sub-structures, systems, etc. The anatomical structures may be detected according to any known approach. For example, anatomical structures may be detected using regression forests, heatmap regression with convolutional neural networks, or deep reinforcement learning. Figure 4 shows exemplary structures that may be detected in the medical images of the patient population, in accordance with one embodiment. In one embodiment, the anatomical structures may be detected according to workflow 500 of Figure 5, described in further detail below.

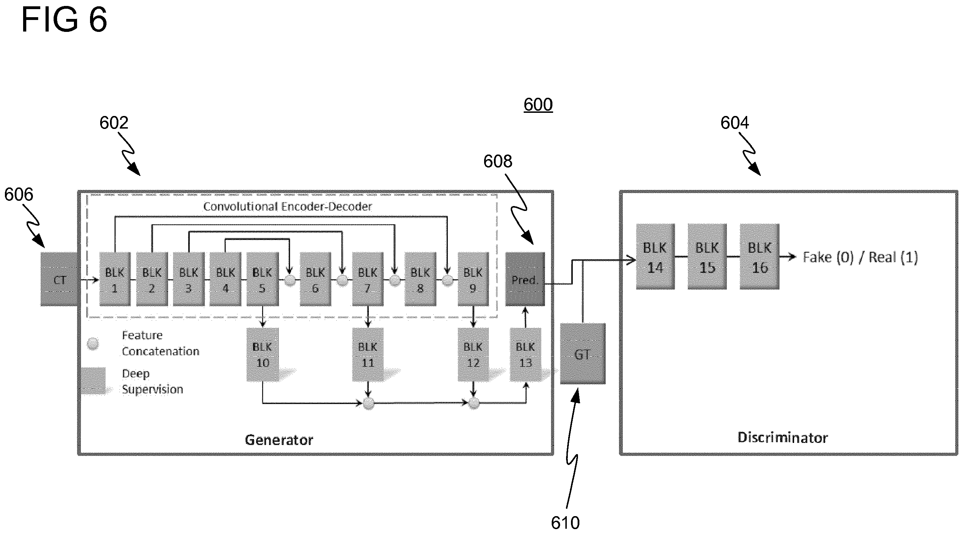

At step 306, one or more of the anatomical structures are segmented from the medical images. The one or more segmented anatomical structures are represented as a segmentation mask having voxels (or pixels, as the case may be) associated with the one or more anatomical structures. The segmentation may be performed using any known approach. For example, the one or more anatomical structures may be segmented using encoder-decoder fully convolutional networks, which may be performed with adversarial training. In one embodiment, the one or more anatomical structures may be segmented using the adversarial deep image-to-image network 600 of Figure 6, described in further detail below.

At step 308, quantitative patient data is determined based on medical records associated with the patient population. The medical records may include, e.g., patient related information (e.g., medical history, demographic information, etc.), medical images reconstructed based on acquisition information (e.g., DICOM information), anatomical structure information (e.g., landmarks, organs, organ sub-structures, systems, etc.) detected in the medical images using a machine learning algorithm, segmented anatomical structures generated using a machine learning algorithm, or any other suitable medical record.

The quantitative patient data may include any quantitative measure determined from the medical records associated with the patient population, such as, e.g., patient related information, reconstructed medical images (reconstructed based on acquisition information), detected, annotated, or segmented anatomical structures, or a presence, a position, a distance, a diameter, a circumference, a volume, a surface area, a score, etc. associated with the anatomical structures For example, the quantitative patient data may be a distance between two anatomical structures, a distance (e.g., length, width, height, diameter, circumference, etc.) associated with an anatomical structure, a volume of an anatomical structure, or a lesion score associated with an anatomical structure.

At step 310, the detected anatomical structures, the one or more segmented anatomical structures, and the quantitative patient data are output as the structured patient data.

Figure 5 shows a workflow 500 for detecting anatomical structures in a medical image. Workflow 500 may be performed as described in U.S. Patent No. 10,096,107, entitled "Intelligent Medical Image Landmark Detection," the disclosure of which is incorporated herein by reference in its entirety. In workflow 500, an artificial intelligence (Al) agent 502 is trained for each anatomical structure to be detected. During a training stage, agent 502 is trained to iteratively navigate in the medical image. Agent 502 selects from a set of actions that enables movement across three dimensions of image space 504 and across image scale 506 from course to fine. Agent 502 is trained to select the best sequence of actions using reinforcement learning by encouraging actions that gets the agent 502 closer to a target structure (or outside of the image if the structure is not present). Agent 502 is modeled using a deep neural network that learns the mapping between the voxel intensities at the current position to the probability of the actions. Each anatomical structure has a corresponding agent 502, and the detected anatomical structure should be spatially coherent according to the distribution from training data. During the testing phase, applying workflow 500, it was found that one medical imaging volume could be parsed in a couple of seconds on average, thus enabling fast processing of large amounts of medical images.

Figure 6 shows an adversarial deep image-to-image (DI2I) network 600 for segmenting one or more anatomical structures from medical images. Adversarial DI2I network 600 may be implemented for segmenting one or more anatomical structures from medical imaging data as described in U.S. Patent Publication No. 2018/0260957, entitled "Automatic Liver Segmentation using Adversarial Image-to-image Network," the disclosure of which is incorporated herein by reference in its entirety. The segmentation task of adversarial DI2I network 600 is defined as a voxel-wise classification on an input medical image.

As shown in Figure 6, generator 602 receives a 3D CT volume 606 as input and outputs a prediction 608 in the form of a segmentation mask. The segmentation mask may be in the form of a probability map or a binary segmentation mask for an anatomical structure. The probability map indicates a voxelwise probability or likelihood that each voxel belongs to a region of an anatomical structure (e.g., liver). The probability map can be converted to the binary segmentation mask by labeling all voxels with a probability greater than a threshold (e.g., 0.5) as positive (in the region of the anatomical structure) and all voxels with a probability less than the threshold as negative (not in the region of the anatomical structure). Generator 602 comprises an encoder represented by blocks BLK1-BLK4 for encoding volume 606 and a decoder represented by blocks BLK5-BLK9 for decoding (generating) corresponding segmentations. Blocks BLK10-BLK13 provide upscaling and deep supervision. Blocks BLK1-BLK13 represent one or more layers of adversarial DI2I network 600.

Discriminator network 604 is utilized during the training stage. Discriminator network 604 is shows with blocks BLK14-BLK16, each of which include one or more convolutional layers. Prediction 608 (e.g., a binary segmentation mask) is input together with a ground truth segmentation 610 to discriminator network 604. The role of discriminator network 604 is to classify one image as the generated image and the other image as the ground truth image. Training is successful if discriminator network 604 cannot distinguish between the generated image and the ground truth image.

Embodiments of the present invention were experimentally validated using the chronic obstructive pulmonary disease (COPDGene) database, which included clinical information, blood samples, test results, and CT medical images for a patient population of over 10,000 patients. The CT images included nearly 24,000,000 CT images comprising more than 40,000 three dimensional CT volumes. CT images is used to identify potential causes for COPD symptoms such as cough or breathlessness, or other causes that present similarly such as bronchiectasis, fibrosis, infections, or cancer. The COPDGene database include lung lobes and airway segmentations that are used to quantify emphysema, potential thickening of the airways, or air trappings for each patient.

Embodiments of the present invention were applied to process CT images of the COPDGene database to determine and compare measurements relating to the effects of COPD and quantitative measurements from lung CT images. The measurements of the effects of COPD were measured as a percentage of low-attenuation areas in the CT images, measured as areas below a predetermined Hounsfield Units (HU) threshold (such as -950 HU or -900 Hu, denoted as LAA950 and LAA900 respectively) and the measurements from lung CT images were measured as the forced expiratory volume (FEV). Figure 7 shows a scatter plot 702 comparing LAA950 with FEV and a scatter plot 704 comparing LAA900 with FEV.

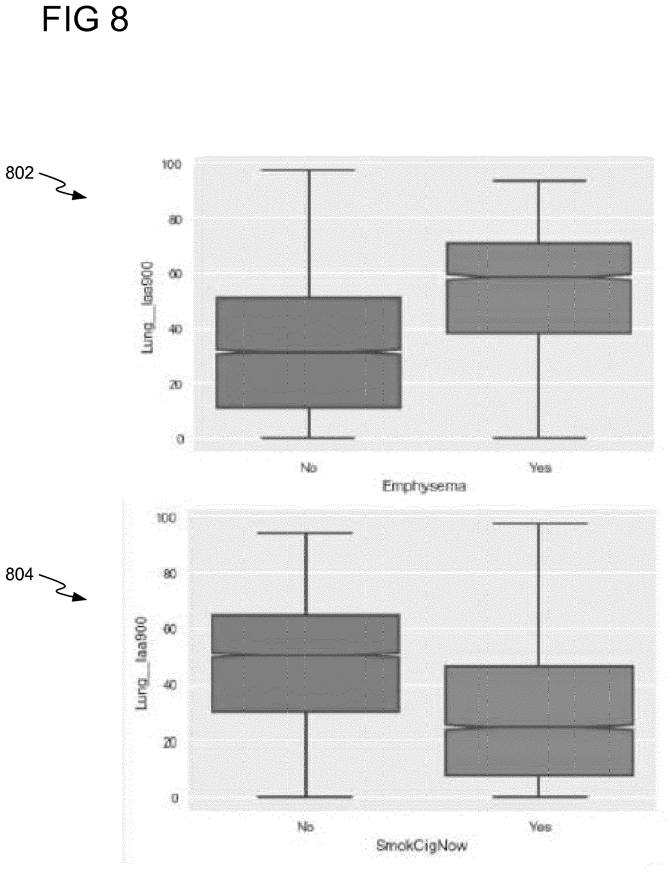

As expected, patients with a current diagnosis for COPD and those with a specific diagnosis for emphysema had a statistically significant (p-value < 10-6) higher percentage of LAA950/LAA900 than those not currently diagnosed. Alternatively, current smokers demonstrated a statistically significant (p-value < 10-6) lower LAA900, or in other words had "denser" lungs than non-current smokers. Figure 8 shows a box plot 802 comparing LAA900 with emphysema diagnosis and a box plot 804 comparing LAA900 with current smoking status. Similar findings have been attributed to the presence of cellular inflammation components in lung smokers, likely in reaction to chronic irritation by smoke.

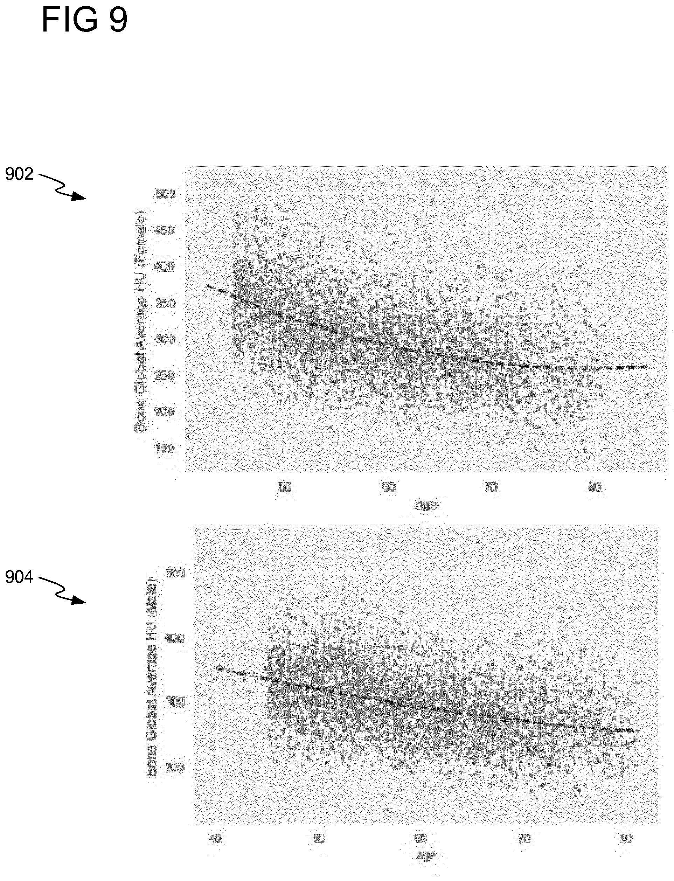

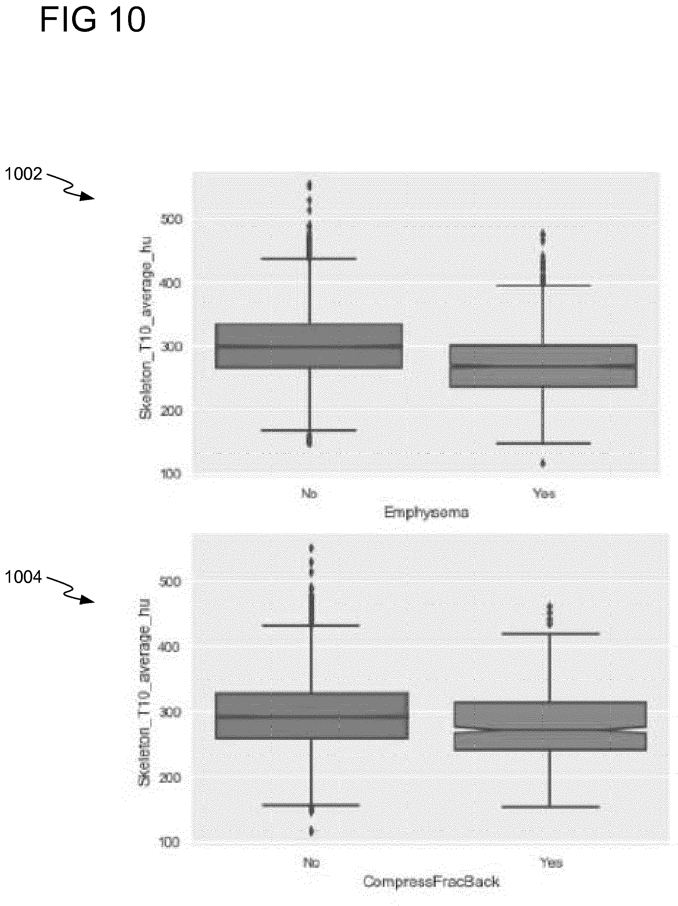

With the automatic processing of a vast number of quantitative values from medical images enabled by embodiments of the present invention, quantities considered unrelated to the pathology of interest can be analyzed for opportunistic research studies or diagnostics. For example, images collected for the study of a primarily respiratory disease (COPD) were utilized to perform an unrelated skeletal tissue analysis: the study of bone mineral density (BMD) globally or of specific vertebrae in relation to age. When considering bone mineral density, a decrease in bone density was demonstrated with age for both male and female patients, albeit at different rates of decrease. Figure 9 shows a scatter plot 902 comparing global bone density for female patients with age and a scatter plot 904 comparing global bone density for male patients with age. This corresponds to the expected relationship between BMD and age based on current knowledge. Also as expected, patients with a history of compression fractures had a statistically significant (p-value < 10-6) lower BMD than those without. Interestingly, it was also demonstrated that patients with medically diagnosed chronic bronchitis has a statistically significant (p-value < 10-6) lower body mass index (BMI) than patients diagnosed with emphysema. Figure 10 shows a box plot 1002 comparing BMI with emphysema diagnosis and a box plot 1004 comparing BMI with a current diagnosis of compression factures.

Embodiments of the present invention were experimentally shown to be able to quickly analyze complex relationships between such quantitative data and clinical data. A similar study of 10,000 patients using conventional manual data collection and bone segmentation would likely take months, however this analysis using embodiments of the present invention took one day. Embodiments of the present invention enable the automatic transformation of raw data into new and actionable information that can be used for planning, diagnosis, decision support, and intervention.

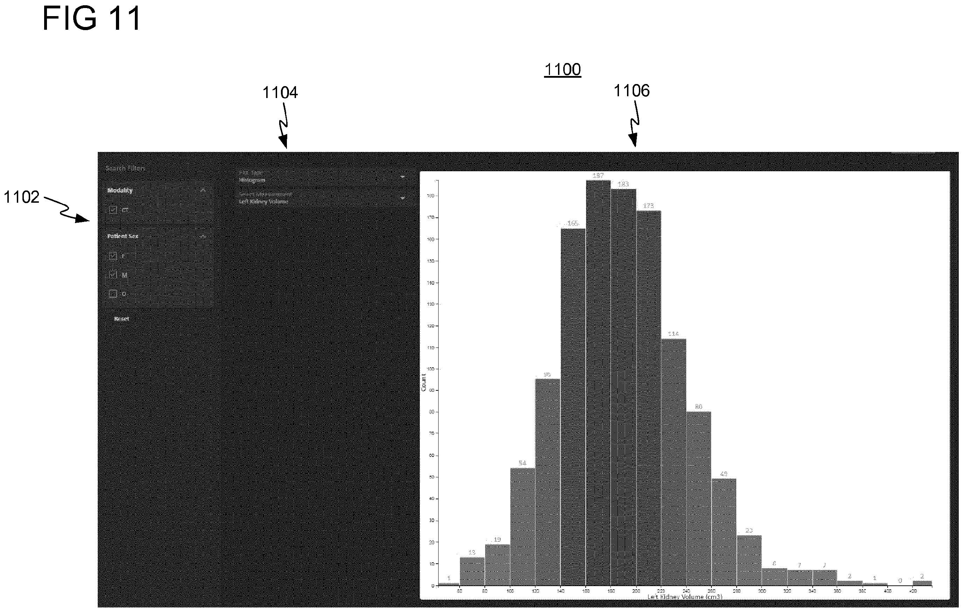

Figure 11 shows a user interface 1100 for interacting with patient data analysis system 106 of Figure 1, in accordance with one or more embodiments. User interface 1100 may be a user interface of computing device 102 of Figure 1 in one embodiment. A user may interact with user interface 1100 to define filters 1102 and analytic measure types 1104, e.g., as parameters of the request at step 204 of Figure 2. Filters 1102 are for filtering structured patient data by modality (e.g., CT) or patient sex (e.g., female, male, other). Analytic measure type 1104 are for defining the type of the analytic measure, such as, e.g., plot type (e.g., histogram) and the quantitative patient data (e.g., left kidney volume). User interface 1100 shows a histogram 1106 as the analytic measure. Histogram 1106 compares left kidney volume with number of patients.

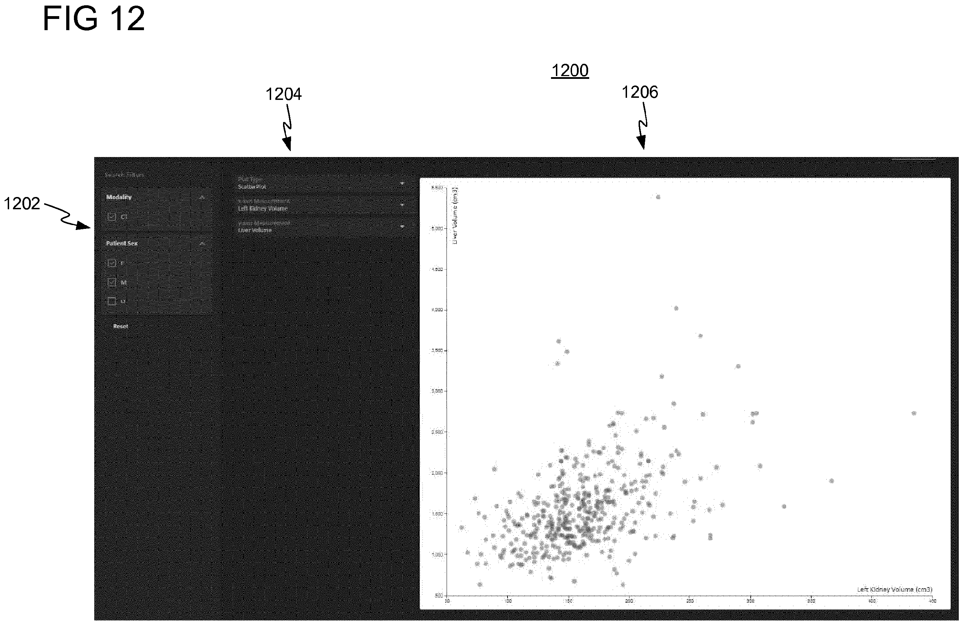

Figure 12 shows a user interface 1200 for interacting with patient data analysis system 106 of Figure 1, in accordance with one or more embodiments. User interface 1200 may be a user interface of computing device 102 of Figure 1 in one embodiment. A user may interact with user interface 1200 to define filters 1202 and analytic measure types 1204, e.g., as parameters of the request at step 204 of Figure 2. Filters 1202 are for filtering structured patient data by modality (e.g., CT) or patient sex (e.g., female, male, other). Analytic measure type 1204 are for defining the type of the analytic measure, such as, e.g., plot type (e.g., scatter plot), the x-axis measurement (e.g., left kidney volume), and the y-axis measurement (e.g., liver volume). User interface 1200 shows a scatter plot 1206 as the analytic measure. Scatter plot 1206 compares left kidney volume with liver volume.

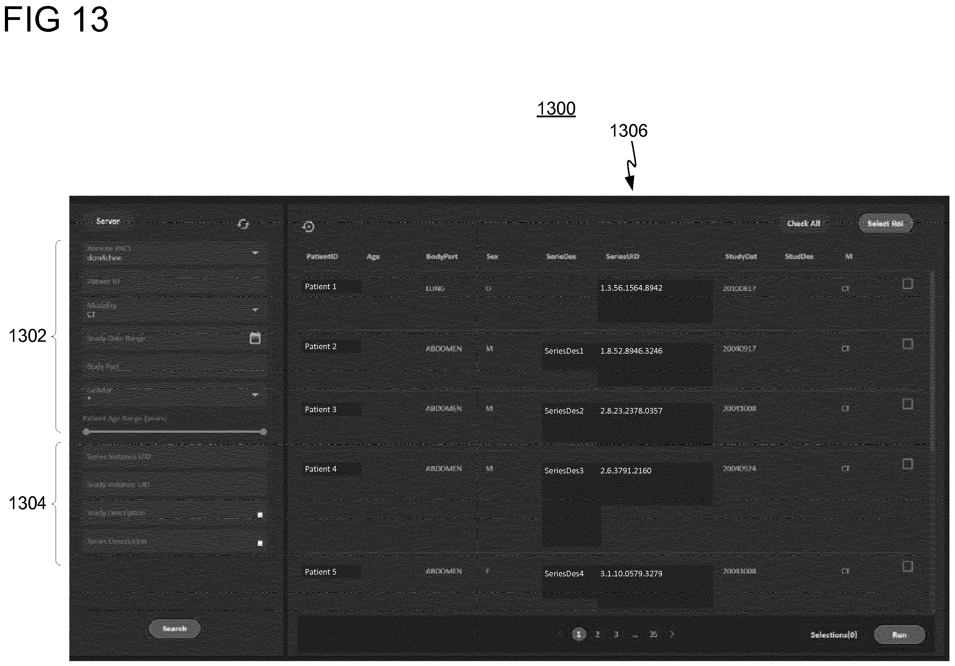

Figure 13 shows a user interface 1300 for interacting with patient data analysis system 106 of Figure 1, in accordance with one or more embodiments. User interface 1300 may be a user interface of computing device 102 of Figure 1 in one embodiment. A user may interact with user interface 1300 to define filters 1302 and display options 1304, e.g., as parameters of the request at step 204 of Figure 2. Filters 1302 are for filtering structured patient data by data source (e.g., PACS name), patient ID, modality (e.g., CT), date range, body part, gender, and age range. Display options 1304 provide options for displaying data, such as, e.g., series instance UID, study instance UID, study description, and series description. User interface 1300 shows a table 1306 as the analytic measure. Table 1306 shows structured data according to filters 1302 and display options 1304.

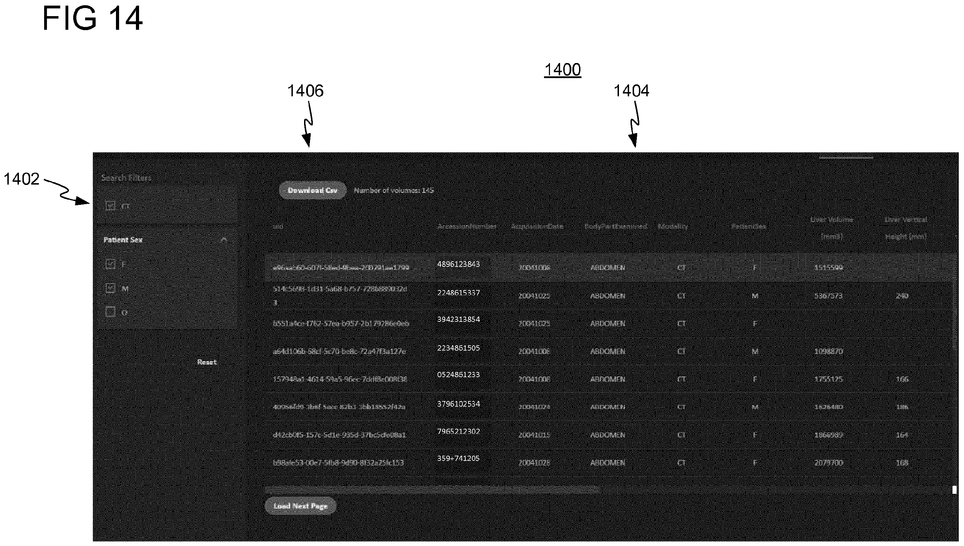

Figure 14 shows a user interface 1400 for interacting with patient data analysis system 106 of Figure 1, in accordance with one or more embodiments. User interface 1400 may be a user interface of computing device 102 of Figure 1 in one embodiment. A user may interact with user interface 1400 to define filters 1402, e.g., as parameters of the request at step 204 of Figure 2. Filters 1402 are for filtering structured patient data by modality (e.g., CT) and patient sex (e.g., male, female, other). User interface 1400 shows a table 1404 as the analytic measure. Table 1404 shows structured data according to filters 1402. User interface 1400 includes a download button 1408 for exporting the structured data in a standardized format, such as, e.g., a comma-separated values (CSV) format as shown in Figure 14. The exported structured data may be used as input to other analytic systems or tools for deeper analysis.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 2-3. Certain steps or functions of the methods and workflows described herein, including one or more of the steps or functions of Figures 2-3, may be performed by a server or by another processor in a network-based cloud-computing system. Certain steps or functions of the methods and workflows described herein, including one or more of the steps of Figures 2-3, may be performed by a client computer in a network-based cloud computing system. The steps or functions of the methods and workflows described herein, including one or more of the steps of Figures 2-3, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 2-3, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

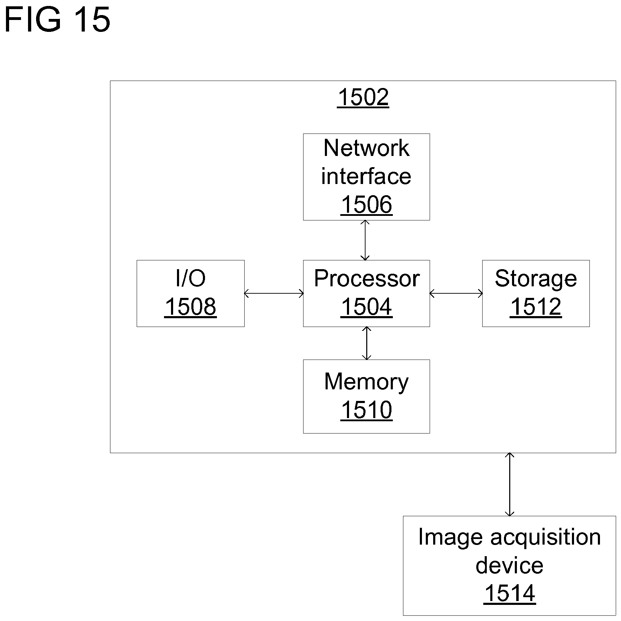

A high-level block diagram of an example computer 1502 that may be used to implement systems, apparatus, and methods described herein is depicted in Figure 15. Computer 1502 includes a processor 1504 operatively coupled to a data storage device 1512 and a memory 1510. Processor 1504 controls the overall operation of computer 1502 by executing computer program instructions that define such operations. The computer program instructions may be stored in data storage device 1512, or other computer readable medium, and loaded into memory 1510 when execution of the computer program instructions is desired. Thus, the method and workflow steps or functions of Figures 2-3 can be defined by the computer program instructions stored in memory 1510 and/or data storage device 1512 and controlled by processor 1504 executing the computer program instructions. For example, the computer program instructions can be implemented as computer executable code programmed by one skilled in the art to perform the method and workflow steps or functions of Figures 2-3. Accordingly, by executing the computer program instructions, the processor 1504 executes the method and workflow steps or functions of Figures 2-3. Computer 1504 may also include one or more network interfaces 1506 for communicating with other devices via a network. Computer 1502 may also include one or more input/output devices 1508 that enable user interaction with computer 1502 (e.g., display, keyboard, mouse, speakers, buttons, etc.).

Processor 1504 may include both general and special purpose microprocessors, and may be the sole processor or one of multiple processors of computer 1502. Processor 1504 may include one or more central processing units (CPUs), for example. Processor 1504, data storage device 1512, and/or memory 1510 may include, be supplemented by, or incorporated in, one or more application-specific integrated circuits (ASICs) and/or one or more field programmable gate arrays (FPGAs).

Data storage device 1512 and memory 1510 each include a tangible non-transitory computer readable storage medium. Data storage device 1512, and memory 1510, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 1508 may include peripherals, such as a printer, scanner, display screen, etc. For example, input/output devices 1508 may include a display device such as a cathode ray tube (CRT) or liquid crystal display (LCD) monitor for displaying information to the user, a keyboard, and a pointing device such as a mouse or a trackball by which the user can provide input to computer 1502.

An image acquisition device 1514 can be connected to the computer 1502 to input image data (e.g., medical images) to the computer 1502. It is possible to implement the image acquisition device 1514 and the computer 1502 as one device. It is also possible that the image acquisition device 1514 and the computer 1502 communicate wirelessly through a network. In a possible embodiment, the computer 1502 can be located remotely with respect to the image acquisition device 1514.

Any or all of the systems and apparatus discussed herein, including elements of computing device 102, patient data analysis system 106, knowledge database 108, medical imaging database 110, and electronic medical records 112 of Figure 1, may be implemented using one or more computers such as computer 1502.

One skilled in the art will recognize that an implementation of an actual computer or computer system may have other structures and may contain other components as well, and that Figure 15 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

## Claims

1. A method comprising:
maintaining (202) a knowledge database comprising structured patient data for a patient population, the structured patient data generated by processing unstructured medical imaging data for the patient population using one or more machine learning algorithms;
determining (206) an analytic measure of the patient population based on the structured patient data of the knowledge database; and
outputting (208) the analytic measure of the patient population.

2. The method according to claim 1, wherein the structured patient data comprises anatomical structures, one or more segmented anatomical structures, and quantitative patient data.

3. The method according to claim 2, wherein processing unstructured medical imaging data for the patient population using one or more machine learning algorithms comprises:
detecting (304) the anatomical structures in medical images of the unstructured medical imaging data for the patient population;
segmenting (306) the one or more of the anatomical structures from the medical images; and
determining (308) the quantitative patient data based on medical records of the patient population,
and/or
wherein the quantitative patient data comprises at least one of a presence, a position, a distance, a diameter, a circumference, a volume, a surface area, and a score.

4. The method according to any of the preceding claims, wherein determining an analytic measure of the patient population based on the structured patient data of the knowledge database comprises:
generating a graph or a table based on the structured patient data.

5. The method according to any of the preceding claims, wherein the determining the analytic measure of the patient population is in response to a user request, in particular, wherein the user request comprises a query for filtered structured patient data from the knowledge database.

6. The method according to any of the preceding claims, wherein the unstructured medical imaging data comprises medical images of the patient population and medical imaging reports of the medical images.

7. The method according to any of the preceding claims, wherein outputting the analytic measure of the patient population comprises displaying the analytic measure of the patient population on a display device
or
wherein outputting the analytic measure of the patient population comprises exporting the analytic measure to a file.

8. An apparatus, comprising:
means for maintaining (202) a knowledge database comprising structured patient data for a patient population, the structured patient data generated by processing unstructured medical imaging data for the patient population using one or more machine learning algorithms;
means for determining (206) an analytic measure of the patient population based on the structured patient data of the knowledge database; and
means for outputting (208) the analytic measure of the patient population.

9. The apparatus according to claim 8, wherein the structured patient data comprises anatomical structures, one or more segmented anatomical structures, and quantitative patient data.

10. The apparatus according to claim 9, wherein processing unstructured medical imaging data for the patient population using one or more machine learning algorithms comprises:
means for detecting (304) the anatomical structures in medical images of the unstructured medical imaging data for the patient population;
means for segmenting (306) the one or more of the anatomical structures from the medical images; and
means for determining (308) the quantitative patient data based on medical records of the patient population.

11. The apparatus according to any of the preceding claims 9 or 10, wherein the quantitative patient data comprises at least one of a presence, a position, a distance, a diameter, a circumference, a volume, a surface area, and a score.

12. A non-transitory computer readable medium storing computer program instructions, the computer program instructions when executed by a processor cause the processor to perform operations comprising:
maintaining (202) a knowledge database comprising structured patient data for a patient population, the structured patient data generated by processing unstructured medical imaging data for the patient population using one or more machine learning algorithms;
determining (206) an analytic measure of the patient population based on the structured patient data of the knowledge database; and
outputting (208) the analytic measure of the patient population.

13. The non-transitory computer readable medium according to claim 12, wherein the structured patient data comprises anatomical structures, one or more segmented anatomical structures, and quantitative patient data, and wherein processing unstructured medical imaging data for the patient population using one or more machine learning algorithms comprises:
detecting (304) the anatomical structures in medical images of the unstructured medical imaging data for the patient population;
segmenting (306) the one or more of the anatomical structures from the medical images; and
determining (308) the quantitative patient data based on medical records of the patient population.

14. The non-transitory computer readable medium according to any of the preceding claims 12 or 13, wherein determining an analytic measure of the patient population based on the structured patient data of the knowledge database comprises:
generating a graph or a table based on the structured patient data,
or
wherein the determining the analytic measure of the patient population is in response to a user request.

15. The non-transitory computer readable medium according to any of the preceding claims 12 to 14, wherein the unstructured medical imaging data comprises medical images of the patient population and medical imaging reports of the medical images.

16. The non-transitory computer readable medium according to any of the preceding claims 12 to 15, wherein outputting the analytic measure of the patient population comprises displaying the analytic measure of the patient population on a display device.
